(19) Europäisches Patentamt
European Patent Office
Office européen des brevets

(11) **EP 3 825 926 A1**

(12) **EUROPEAN PATENT APPLICATION**

(43) Date of publication:
**26.05.2021 Bulletin 2021/21**

(51) Int Cl.:
**G06N 5/04** *(2006.01)*          **G06N 20/00** *(2019.01)*
**G16H 50/30** *(2018.01)*          **A61B 5/00** *(2006.01)*

(21) Application number: **20150307.5**

(22) Date of filing: **06.01.2020**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
**KH MA MD TN**

(30) Priority: **19.11.2019 US 201962937435 P**

(71) Applicant: **Koninklijke Philips N.V.
5656 AG Eindhoven (NL)**

(72) Inventors:
• **Dong, Junzi**
  **5656 AE Eindhoven (NL)**
• **Conroy, Bryan**
  **5656 AE Eindhoven (NL)**

(74) Representative: **Steenbeek, Leonardus Johannes
et al
Philips Intellectual Property & Standards
High Tech Campus 5
5656 AE Eindhoven (NL)**

(54) **PERSONALIZED INTERPRETATION OF FEATURE IMPORTANCE FOR MACHINE LEARNING MODELS**

(57)    A method for determining feature importances of two or more features in a machine learning model in which feature-specific functions are applied to the two or more features to obtain an output of the machine learning model, the method comprising: receiving a sample; computing for two or more features of the received sample, the feature importance in dependence on the feature-specific functions applied to the feature of the received sample and a respectively corresponding reference value; selecting one or more features based upon absolute values of the feature importances; and sending the selected one or more features to a display.

FIG. 4

EP 3 825 926 A1

## Description

### TECHNICAL FIELD

Various exemplary embodiments disclosed herein relate generally to personalized interpretation of feature importance for machine learning models.

### BACKGROUND

Understanding why a prediction is made is crucial to building trust in machine learning models among users, and ultimately influences whether a model will be adapted or shelved. This is especially true in the healthcare industry. Previous work in this area such as partial dependence plots have focused on global feature importance and not the interpretation of individual predictions. Though helpful in understanding how models work overall, global feature importance does not provide personalized interpretation of why individual predictions are made.

### SUMMARY

It is an object of the invention to provide a personalized interpretation of feature importance for machine learning models. The invention is defined by the independent claims; the dependent claims define advantageous embodiments. A summary of various exemplary embodiments is presented below. Some simplifications and omissions may be made in the following summary, which is intended to highlight and introduce some aspects of the various exemplary embodiments, but not to limit the scope of the invention. Detailed descriptions of an exemplary embodiment adequate to allow those of ordinary skill in the art to make and use the inventive concepts will follow in later sections.

Various embodiments relate to a system for determining the importance of features in a machine learning model, including: a memory; a processor connected to the memory, the processor configured to: train a generalized additive model (GAM); calculate the normal population value for each feature; receive a sample; compute the feature importance (FI) for each feature of the received sample; rank the FI for each feature by absolute value; and determine the top features based upon the FI rankings; and send the determined top features to a display.

Various embodiments are described, further including: a display with a graphical user interface configured to display the top features for the received sample.

Various embodiments are described, wherein processor is further configured to: compute the global feature importance for each feature; rank the global feature importance for each feature by absolute value; determine the top global features based upon the global feature importance rankings; and send the determined top global features to a display.

Various embodiments are described, wherein the GAM is defined as $y = \beta_0 + f_1(x_1) + f_2(x_2) + \cdots + f_m(x_m)$, where y is the output of the GAM, $\beta_0$ is an bias value, $f_1$, $f_2$, $\cdots$ $f_m$ are set of functions independently applied to each of the features $x_1$, $x_2$, $\cdots$ $x_m$ respectively.

Various embodiments are described, wherein FI is computed as follows:

$$FI_i = f_i(x_i) - f_i(\bar{x}_i),$$

where $f_i(\ )$ is the feature-specific predictor function from the GAM, $x_i$ is the value of the $i$th feature for the received sample, and $\bar{x}_i$ is the normal population value of the $i$th feature.

Various embodiments are described, wherein the normal population value is one of a mean value, median value, or weighted mean value of the sample population for each feature.

Various embodiments are described, wherein the FI value is weighted by another GAM variable.

Various embodiments are described, wherein the number of top features to display is based upon a predetermined number of top features and/or a threshold for the magnitude of the FI values.

Further various embodiments relate to a method for determining the importance of features in a machine learning model, including: training, by a processor, a generalized additive model (GAM); calculating the normal population value for each feature; receiving a sample; computing the feature importance (FI) for each feature of the received sample; ranking the FI for each feature by absolute value; and determining the top features based upon the FI rankings; and sending the determined top features to a display.

Various embodiments are described, further including: displaying, by a display, the top features for the received sample.

Various embodiments are described, further including: computing the global feature importance for each feature;

ranking the global feature importance for each feature by absolute value; determining the top global features based upon the global feature importance rankings; and sending the determined top global features to a display.

Various embodiments are described, wherein the GAM is defined as $y = \beta_0 + f_1(x_1) + f_2(x_2) + \cdots + f_m(x_m)$, where y is the output of the GAM, $\beta_0$ is an bias value, $f_1$, $f_2$, ... $f_m$ are set of functions independently applied to each of the features $x_1$, $x_2$, $\cdots x_m$ respectively.

Various embodiments are described, wherein FI is computed as follows:

$$FI_i = f_i(x_i) - f_i(\bar{x}_i),$$

where $f_i(\ )$ is the feature-specific predictor function from the GAM, $x_i$ is the value of the $i$th feature for the received sample, and $\bar{x}_i$ is the normal population value of the $i$th feature.

Various embodiments are described, wherein the normal population value is one of a mean value, median value, or weighted mean value of the sample population for each feature.

Various embodiments are described, wherein the FI value is weighted by another GAM variable.

Various embodiments are described, wherein the number of top features to display is based upon a predetermined number of top features and/or a threshold for the magnitude of the FI values.

**BRIEF DESCRIPTION OF THE DRAWINGS**

In order to better understand various exemplary embodiments, reference is made to the accompanying drawings, wherein:

FIG. 1 is an example plot showing global feature importance, as calculated using normalized coefficients of the linear regression model described in an example of predicting risk of hemodynamic instability using adaptive boosting;

FIG. 2 illustrates an example of a univariate classifier $f_i(x_i)$ for non-invasive mean blood pressure;

FIG. 3 illustrates two examples of individual feature importance in blood gas prediction. In each example, the predicted pH is shown at the top;

FIG. 4 illustrates another example of how feature importance may be displayed; and

FIG. 5 illustrates an exemplary hardware diagram for implementing a feature interpretation system.

To facilitate understanding, identical reference numerals have been used to designate elements having substantially the same or similar structure and/or substantially the same or similar function.

**DETAILED DESCRIPTION OF EMBODIMENTS**

The description and drawings illustrate the principles of the invention. It will thus be appreciated that those skilled in the art will be able to devise various arrangements that, although not explicitly described or shown herein, embody the principles of the invention and are included within its scope. Furthermore, all examples recited herein are principally intended expressly to be for pedagogical purposes to aid the reader in understanding the principles of the invention and the concepts contributed by the inventor(s) to furthering the art and are to be construed as being without limitation to such specifically recited examples and conditions. Additionally, the term, "or," as used herein, refers to a non-exclusive or (*i.e.*, and/or), unless otherwise indicated (*e.g.,* "or else" or "or in the alternative"). Also, the various embodiments described herein are not necessarily mutually exclusive, as some embodiments can be combined with one or more other embodiments to form new embodiments.

Embodiments of a method are disclosed herein for interpreting individual predictions made by a basic class of machine learning models-generalized additive models (GAMs). This method enables identifying the most important input features to be recommended along with the prediction and will help in improving the interpretability, trust-worthiness, and safety of machine learning models.

GAMs are a class of machine learning models where the prediction may be expressed as the linear sum of independent predictor functions of individual inputs. With $m$ input features $x_1$, $x_2$, $\cdots x_m$ predicting outcome $y$, the GAM may be expressed as:

$$y = \beta_0 + f_1(x_1) + f_2(x_2) + \cdots + f_m(x_m),$$

where $\beta_0$ is a bias or offset value, and $f_1$, $f_2$, $\cdots f_m$ are a set of functions independently applied to each of the features

$x_1, x_2, \cdots x_m$. The functions $f_1, f_2, \cdots f_m$ may take various forms and may be selected based upon the specific feature and its known relationship to the output $y$. Both $\beta_0$ and parameters defining the functions $f_1, f_2, \cdots f_m$ may be determined using training data and machine learning techniques.

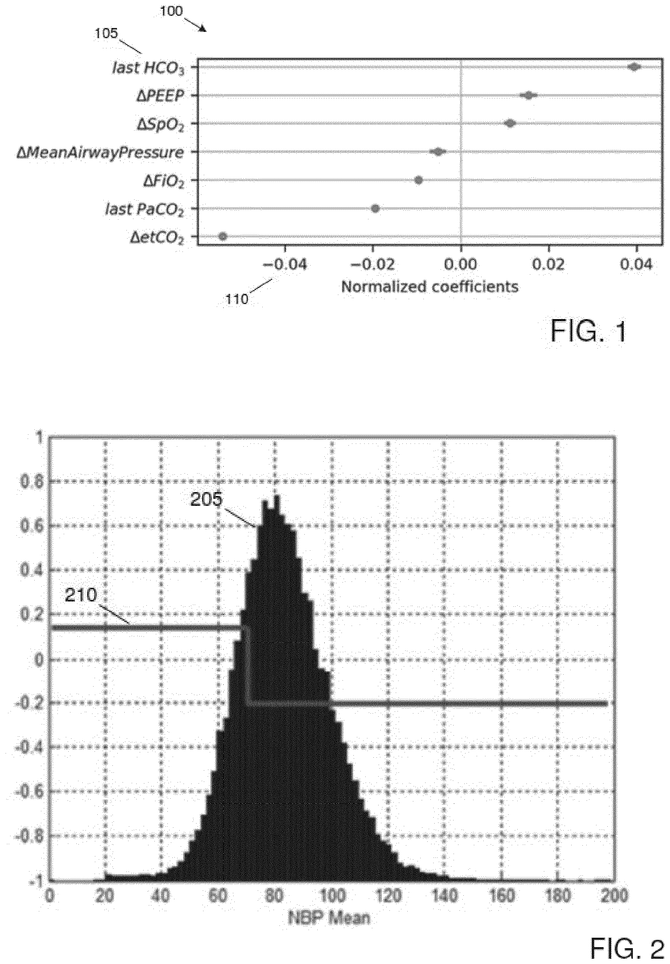

GAMs are often used for clinical decision support (CDS) algorithms due to their simplicity and interpretability. However, models are typically interpreted as a whole, that is for all predictions, not on the level of individual predictions. Global interpretation takes some form of feature ranking, where the order of feature importance is presented to users, who can check to make sure that global feature rankings match their intuition. However, for specific predictions, the impact of individual features varies, and global feature importance does not explain why a specific prediction is made. FIG. 1 is an example plot showing global feature importance, as calculated using normalized coefficients of the linear regression model described in an example of predicting risk of hemodynamic instability using adaptive boosting discussed below. The plot 100 has a vertical axis with a list of features 105 ordered based upon the value of normalized coefficients. The horizontal axis indicates the normalized coefficient values 110. As can be seen, a point is plotted for each feature based upon the associated normalized coefficient. Features with normalized coefficients close to zero have less influence on the output, where features that have normalized features with greater magnitude have more influence on the output value. In this example, the features $\Delta etCO_2$ and last $HCO_3$ have the greatest influence on the output. In the case of $\Delta etCO_2$, the output is reduced, and for $HCO_3$, the output is increased. While providing some interpretability of the algorithm as a whole, it is difficult to explain individual predictions using this display of global feature importance. Knowing which input features impact a specific prediction may give users real-time information about how the algorithm arrives at a prediction, allowing users to match predictions with their intuition on a more intuitive case-by-case basis.

An embodiment of a method for highlighting the feature importance for individual predictions will now be described, which will enable human users to compare the prediction mechanism with their own intuition for individual prediction cases.

The method includes the calculation of case-specific feature importance (FI) scores, which may then be ranked to display the features with most impact on a specific prediction. The FI for the $i$th feature of a specific sample $x$ is expressed as:

$$FI_i = f_i(x_i) - f_i(\bar{x}_i),$$

where $f_i(\ )$ is the feature-specific predictor function from the GAM, $x_i$ is the value of the ith feature for this sample, and $\bar{x}_i$ is, in this example, the normal population mean of the $i$ th feature. In other embodiments, $\bar{x}_i$ may be determined in other ways, such as for example the median, weighted mean, etc.

Here, $\bar{x}_i$ represents a typical uninformative background value (that is, it is informative as to the group, but not to specific predictions), and $f_i(\bar{x}_i)$ is the typical uninformative feature-specific predictor value. When $x_i$ is close to the population mean $\bar{x}_i$, $FI_i$ will be equal or close to 0, which means feature $i$ contributes little to the overall prediction of sample $\vec{x}$. When $x_i$ deviates from the normal population mean, $FI_i$ shifts away from 0 to highlight the contribution of the feature at hand to the prediction.

$FI$ may be positive or negative, with a larger absolute value indicating more importance. When the prediction task is a binary classification of 0 or 1, the signage of $FI$ represents elevated or decreased probability of binary class 1 contributed by the feature value as compared to the population mean. For example, if $y$ is being predicted, the clinical risk of a patient developing heart failure, positive $FI$ indicates higher risk, while negative $FI$ indicates that a feature is protective, i.e., effectively lowering the risk of heart failure for this patient. When the prediction is a regression problem and the predicted variable $y$ represents a real value, signage represents whether the feature value leads to an increase or decrease in predicted variable $y$, as compared to a feature value of population mean. In both cases, features with the largest absolute value of $FI$ are most important.

As previously described, this method of personalized feature importance ranking may be used for any GAM. The steps for computing personalized feature importance will first be described, and then two examples of application in the healthcare domain will be described.

The individual feature importance score can be calculated with the following steps:

1. Train the GAM

$$y = \beta_0 + f_1(x_1) + f_2(x_2) + \cdots + f_m(x_m)$$

2. Calculate the normal population mean for each feature:

$$[\bar{x}_1, \bar{x}_2, \cdots, \bar{x}_m]$$

3. For a specific data sample *x*, compute FI for each feature:

$$[FI_1(x), FI_2(x), \cdots, FI_m(x)]$$

4. Rank [$FI_1(x)$, $FI_2(x)$, $\cdots$, $FI_m(x)$] by absolute value and display top features to clinicians

Training of the GAM model may be done using labelled training data, various types of function models, and typical machine learning training methods. In additional to calculating the mean for each feature, a median value, weighted mean, or other method for determining the typical value for the feature may be used. Once the FI values are ranked, they may be further analyzed and/or displayed to a user for further evaluation. The number of features displayed may be based on a predetermined number, such as for example, the top 4 features. Alternatively, the features with an FI that exceed a predetermined threshold value may be shown, for example a features with an FI having a magnitude greater than 0.1. Also, a threshold value and a limit to the number of features may both be used to determine how may features to display. The global importance values for each feature may also be presented to the user, so that the user may be able to detect any anomalies in the FI values that do not make sense and hence may either be discounted or further investigated based upon the user's determination.

In addition to being used for prediction interpretation, *FI* may also be used or modified to assess prediction quality in models that allow missing values in input features. An additional input quality model may be developed, using *FI,* feature missingness, and global feature importance, to assess prediction quality. If the highest ranked features by FI are those with missing features, and ranking by FI differs from ranking of global importance (compared using correlation coefficients or manual methods), then the sample is likely of low prediction quality due to too many missing inputs.

Depending on what the end users want to visualize, *FI* may be weighted by other variables to highlight certain predictions over others. For example, when predicting risk of heart failure, a user may only want to highlight top risk features when the total risk of heart failure is elevated. In that case, the FI may be multiplied with the total risk before visualization.

A first example of predicting the risk of hemodynamic instability using adaptive boosting will now be described. Hemodynamic instability (HI) occurs when a patient's circulatory system is unable to meet the metabolic demands of the body, and HI may lead to severe deterioration in health and death. A model was developed to predict whether a patient is at risk for hemodynamic instability, using univariate adaptive boosting, which may be classified as another type of GAM.

The model predicts *y*, the risk of HI, by combining univariate classifiers $f_i(x_i)$ of individual risk contributed by single features. FIG. 2 illustrates an example of a univariate classifier $f_i(x_i)$ for non-invasive mean blood pressure (NMBP). The histogram plot 205 is an underlay showing the distribution of the patient population by NMBP. The line 210 is the value of the univariate classifier risk for a given NMBP input value. The predictions *y* is the sum of all univariate risks.

In the calculation of *FI* for this one feature, it is desired to highlight cases where HI risk is higher (because lower blood pressure is bad for HI), so *FI* is multiplied by 1 - y. In this case:

$$FI_i = [f_i(x_i) - f_i(\bar{x}_i)] \cdot [1 - y(\vec{x})]$$

In FIG. 2, NMBP has only one decision boundary of 70.5; as a result, there are only three scenarios for NMBP measurements: below 70.5, above 70.5, and blood pressure not measured. Table 1 shows the *FI* for these three ranges in NMBP input value, and for two scenarios of predicted y.

Table 1

| NMBP | $f_i(x_i)$ | $f_i(\bar{x}_i)$ | y | $FI_i$ |
|---|---|---|---|---|
| < 70.5 | 0.14 | -0.21 | | 0.315 |
| ≥70:5 | -0.21 | -0.21 | 0.1 | 0 |
| Not measured | 0 | No adjustment | | 0 |
| < 70.5 | 0.14 | -0.21 | | 0.035 |

(continued)

| NMBP | $f_i(x_i)$ | $f_i(\overline{x_i})$ | y | $FI_i$ |
|---|---|---|---|---|
| ≥70:5 | -0.21 | -0.21 | 0.9 | 0 |
| Not measured | 0 | No adjustment | | 0 |

In the first case for y = 0.1, it is noted that $FI_i$ is 0.315 for NMBP < 70.5 and 0 otherwise. In the second case for y = 0.9, it is noted that $FI_i$ is 0.035 for NMBP < 70.5 and 0 otherwise. Because of the weighting $[1 - y(\vec{x})]$, when the output is 0.1, the feature $\overline{x_i}$ affects the output y more than when the output is 0.9.

A second example of predicting blood gas pH using linear regression will now be described.

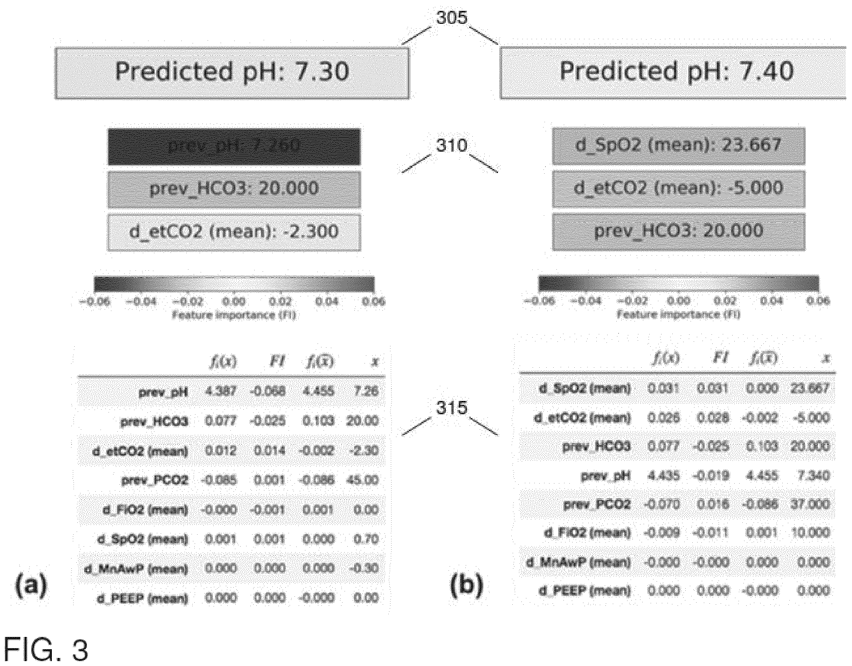

FIG. 3 illustrates two examples of individual feature importance in blood gas prediction. In each example, the predicted pH 305 is shown at the top. The middle portion displays the top three features and their input values 310, color-coded by FI. The bottom table shows how the FI for each feature was calculated.

Blood gas pH is an invasive measurement taken frequently in patients with respiratory distress, sometimes as often as every hour. The amount of blood loss and potential adverse outcomes associated with invasive blood draws motivated the development of a non-invasive blood gas prediction algorithm. In this algorithm, a linear regression was used (a type of GAM) to predict pH using current non-invasive measurements and previous invasive measurements. The use of this algorithm will enable clinicians to delay or avoid some blood draws.

Two examples of applying the individual feature importance method on pH prediction will be shown. In FIG. 3, each example shows the predicted pH 305 and a table 315 including the FI of each feature ranked by contribution to the prediction and how FI values were calculated. In FIG. 3a, the previous pH and previous bicarbonate (HCO3) contribute to lower pH predictions compared to the population norms, while the change in end-tidal CO2 (d_etCO2) contributes to increased predicted pH. d_etCO2 is the amount of CO2 expired by a patient, so the negative input value (-2.3) reflects a decrease in CO2 in the patient's blood stream. This most likely reflects better oxygenation, and the algorithm predicts increased pH (7.30) compared to the previous measurement (7.26). In FIG. 3b, there is a similar affect in d_etCO2 and d_SpO2 changes. Here, the change in SpO2 is positive, indicating higher oxygenation, which matches the directional change of d_etCO2. The previous HCO3 reflects that the patient started at a lower oxygenation state (lower than normal pH), but the trend of d_SpO2 and d_etCO2 indicate that the patient has most likely improved. Taken together, the algorithm predicts a close to normal pH.

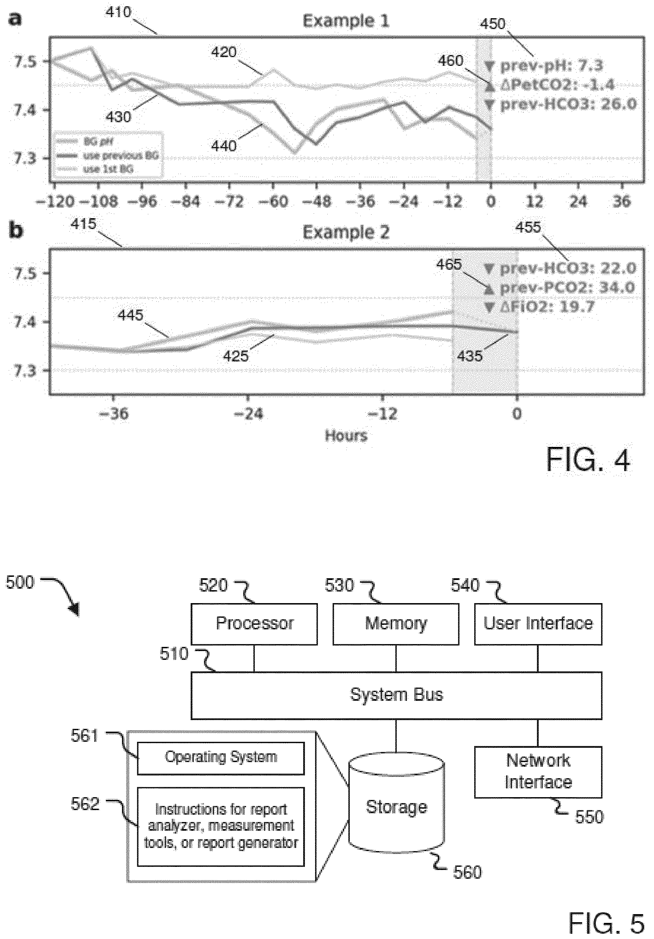

FIG. 4 illustrates another example of how feature importance may be displayed. The plot of blood gas (BG) pH 440, 445 is shown over time. A prediction of the BG pH 430, 435 using the previous BG pH value is shown over time. Also, a prediction of the BG pH 420, 425 using the first BH pH value is shown over time. The top three features 450, 455 for the current time (0) are displayed along with the direction of each feature's contribution 460, 465 (up or down). The signage of FI is used to illustrate the direction of each features contribution, as shown by the up or down arrows 460 and 465. The historic prediction timeline is also displayed for reference. By looking at the predicted value along with the prediction rationale, clinicians may use the top features provided by the individual feature importance method and compare the pH prediction algorithm's intuition with their own clinical understanding.

FIG. 5 illustrates an exemplary hardware diagram 500 for implementing a feature interpretation system. As shown, the device 500 includes a processor 520, memory 530, user interface 540, network interface 550, and storage 560 interconnected via one or more system buses 510. It will be understood that FIG. 5 constitutes, in some respects, an abstraction and that the actual organization of the components of the device 500 may be more complex than illustrated.

The processor 520 may be any hardware device capable of executing instructions stored in memory 530 or storage 560 or otherwise processing data. As such, the processor may include a microprocessor, a graphics processing unit (GPU), field programmable gate array (FPGA), application-specific integrated circuit (ASIC), any processor capable of parallel computing, or other similar devices.

The memory 530 may include various memories such as, for example L1, L2, or L3 cache or system memory. As such, the memory 530 may include static random-access memory (SRAM), dynamic RAM (DRAM), flash memory, read only memory (ROM), or other similar memory devices.

The user interface 540 may include one or more devices for enabling communication with a user and may present information such as that shown in FIGs. 2-4. For example, the user interface 540 may include a display, a touch interface, a mouse, and/or a keyboard for receiving user commands. In some embodiments, the user interface 540 may include a command line interface or graphical user interface that may be presented to a remote terminal via the network interface 550.

The network interface 550 may include one or more devices for enabling communication with other hardware devices. For example, the network interface 550 may include a network interface card (NIC) configured to communicate according to the Ethernet protocol or other communications protocols, including wireless protocols. Additionally, the network interface 550 may implement a TCP/IP stack for communication according to the TCP/IP protocols. Various alternative or additional hardware or configurations for the network interface 550 will be apparent.

The storage 560 may include one or more machine-readable storage media such as read-only memory (ROM), random-access memory (RAM), magnetic disk storage media, optical storage media, flash-memory devices, or similar storage media. In various embodiments, the storage 560 may store instructions for execution by the processor 520 or data upon with the processor 520 may operate. For example, the storage 560 may store a base operating system 561 for controlling various basic operations of the hardware 500. The storage 562 may store instructions for determining the features that contribute most to the outcome for a specific sample as described above.

It will be apparent that various information described as stored in the storage 560 may be additionally or alternatively stored in the memory 530. In this respect, the memory 530 may also be considered to constitute a "storage device" and the storage 560 may be considered a "memory." Various other arrangements will be apparent. Further, the memory 530 and storage 560 may both be considered to be "non-transitory machine-readable media." As used herein, the term "non-transitory" will be understood to exclude transitory signals but to include all forms of storage, including both volatile and non-volatile memories.

While the host device 500 is shown as including one of each described component, the various components may be duplicated in various embodiments. For example, the processor 520 may include multiple microprocessors that are configured to independently execute the methods described herein or are configured to perform steps or subroutines of the methods described herein such that the multiple processors cooperate to achieve the functionality described herein. Further, where the device 500 is implemented in a cloud computing system, the various hardware components may belong to separate physical systems. For example, the processor 520 may include a first processor in a first server and a second processor in a second server.

The feature interpretation system and method described herein helps to improve the interpretability of the results received from a model. These results can help a user to gain confidence in the outcome for a specific model input, based upon the user's experience. This will help in the wider adoption of such CDS systems that use GAM types of models, and in the case of medical applications, it will greatly enhance the care provided to patients and to allow for medical personal to use their time more efficiently to carry out more tasks. Further, the feature interpretation system and method disclosed herein is an improvement over current system that only provide interpretation based upon a global population of samples, which do not provide the specific insights for a single input sample. This will lead to better treatment decisions using CDS systems.

Any combination of specific software running on a processor to implement the embodiments of the invention, constitute a specific dedicated machine.

As used herein, the term "non-transitory machine-readable storage medium" will be understood to exclude a transitory propagation signal but to include all forms of volatile and non-volatile memory.

Although the various exemplary embodiments have been described in detail with particular reference to certain exemplary aspects thereof, it should be understood that the invention is capable of other embodiments and its details are capable of modifications in various obvious respects. As is readily apparent to those skilled in the art, variations and modifications can be affected while remaining within the scope of the invention. Accordingly, the foregoing disclosure, description, and figures are for illustrative purposes only and do not in any way limit the invention, which is defined only by the independent claims.

## Claims

1. A system (500) for determining feature importances of two or more features in a machine learning model in which feature-specific functions are applied to the two or more features to obtain an output of the machine learning model, the system comprising:

   a memory (530);
   a processor (520) connected to the memory (530), the processor configured to:

   receive a sample;
   compute for two or more features of the received sample, the feature importance in dependence on the feature-specific functions applied to the feature of the received sample and a respectively corresponding reference value;
   select one or more features based upon absolute values of the feature importances; and
   send the selected one or more features to a display (540).

2. The system of claim 1, further comprising:

the display (540) with a graphical user interface configured to display the selected features for the received sample.

3. The system of claim 1 or 2, wherein the machine learning model is defined as $y = \beta_0 + f_1(x_1) + f_2(x_2) + \cdots + f_m(x_m)$, where y is the output of the machine learning model, $\beta_0$ is an bias value, and $f_1, f_2, \cdots f_m$ are feature-specific functions independently applied to each of the features $x_1, x_2, \cdots x_m$ respectively.

4. The system of any of the preceding claims, wherein the feature importance FI is computed as follows:

$$FI_i = f_i(x_i) - f_i(\bar{x}_i),$$

where $f_i(\ )$ is the feature-specific predictor function from the machine learning model, $x_i$ is the value of the ith feature for the received sample, and $\bar{x}_i$ is the reference value of the $i$th feature.

5. The system of any of the preceding claims, wherein the reference value is one of a mean value, median value, or weighted mean value of the sample population for each feature.

6. The system of any of the preceding claims, wherein the feature importance value is weighted by another machine learning model variable.

7. The system of any of the preceding claims, wherein the selected features to display are selected based upon a ranking of the feature importances by absolute value, and/or a threshold for the absolute value of the feature importances.

8. A method for determining feature importances of two or more features in a machine learning model in which feature-specific functions are applied to the two or more features to obtain an output of the machine learning model, the method comprising:

receiving a sample;
computing for two or more features of the received sample, the feature importance in dependence on the feature-specific functions applied to the feature of the received sample and a respectively corresponding reference value;
selecting one or more features based upon absolute values of the feature importances; and
sending the selected one or more features to a display (540).

9. The method of claim 8, further comprising:
displaying, by the display (540), the selected features for the received sample.

10. The method of claim 8 or 9, wherein the machine learning model is defined as $y = \beta_0 + f_1(x_1) + f_2(x_2) + \cdots + f_m(x_m)$, where y is the output of the machine learning model, $\beta_0$ is an bias value, and $f_1, f_2, \cdots f_m$ are feature-specific functions independently applied to each of the features $x_1, x_2, \cdots x_m$ respectively.

11. The method of any of the preceding claims 8-10, wherein the feature importance FI is computed as follows:

$$FI_i = f_i(x_i) - f_i(\bar{x}_i),$$

where $f_i(\ )$ is the feature-specific predictor function from the machine learning model, $\bar{x}_i$ is the value of the $i$th feature for the received sample, and $\bar{x}_i$ is the reference value of the ith feature.

12. The method of any of the preceding claims 8-11, wherein the reference value is one of a mean value, median value, or weighted mean value of the sample population for each feature.

13. The method of any of the preceding claims 8-12, wherein the feature importance value is weighted by another machine learning model variable.

14. The method of any of the preceding claims 8-13, wherein the selected features to display are selected based upon a ranking of the feature importances by absolute value, and/or a threshold for the absolute value of the feature importances.

FIG. 1

FIG. 2

305

Predicted pH: 7.30

Predicted pH: 7.40

prev_pH: 7.260

d_SpO2 (mean): 23.667

310

prev_HCO3: 20.000

d_etCO2 (mean): -5.000

d_etCO2 (mean): -2.300

prev_HCO3: 20.000

-0.06  -0.04  -0.02  0.00  0.02  0.04  0.06
Feature importance (FI)

-0.06  -0.04  -0.02  0.00  0.02  0.04  0.06
Feature importance (FI)

315

| | $f_i(x)$ | FI | $f_i(\bar{x})$ | x |
|---|---|---|---|---|
| prev_pH | 4.387 | -0.068 | 4.455 | 7.26 |
| prev_HCO3 | 0.077 | -0.025 | 0.103 | 20.00 |
| d_etCO2 (mean) | 0.012 | 0.014 | -0.002 | -2.30 |
| prev_PCO2 | -0.085 | 0.001 | -0.086 | 45.00 |
| d_FiO2 (mean) | -0.000 | -0.001 | 0.001 | 0.00 |
| d_SpO2 (mean) | 0.001 | 0.001 | 0.000 | 0.70 |
| d_MnAwP (mean) | 0.000 | 0.000 | 0.000 | -0.30 |
| d_PEEP (mean) | 0.000 | 0.000 | -0.000 | 0.00 |

| | $f_i(x)$ | FI | $f_i(\bar{x})$ | x |
|---|---|---|---|---|
| d_SpO2 (mean) | 0.031 | 0.031 | 0.000 | 23.667 |
| d_etCO2 (mean) | 0.026 | 0.028 | -0.002 | -5.000 |
| prev_HCO3 | 0.077 | -0.025 | 0.103 | 20.000 |
| prev_pH | 4.435 | -0.019 | 4.455 | 7.340 |
| prev_PCO2 | -0.070 | 0.016 | -0.086 | 37.000 |
| d_FiO2 (mean) | -0.009 | -0.011 | 0.001 | 10.000 |
| d_MnAwP (mean) | -0.000 | -0.000 | 0.000 | 0.000 |
| d_PEEP (mean) | 0.000 | 0.000 | -0.000 | 0.000 |

(a)

(b)

FIG. 3

FIG. 4

FIG. 5

Europäisches
Patentamt

European
Patent Office

Office européen
des brevets

# EUROPEAN SEARCH REPORT

Application Number

EP 20 15 0307

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2018/220600 A1 (UNIV FLORIDA [US]) 6 December 2018 (2018-12-06) * figures 3-5 * * paragraphs [0010] - [0014] * * paragraphs [0029] - [0036] * * paragraphs [0039], [0040] * * paragraphs [0056] - [0064] * ----- | 1-14 | INV. G06N5/04 G06N20/00 G16H50/30 A61B5/00 |

TECHNICAL FIELDS
SEARCHED       (IPC)

G06N

The present search report has been drawn up for all claims

| Place of search | Date of completion of the search | Examiner |
|---|---|---|
| The Hague | 14 July 2020 | Millet, Guillaume |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

.......................................................................
& : member of the same patent family, corresponding
document

EPO FORM 1503 03.82 (P04C01)

**EP 3 825 926 A1**

**ANNEX TO THE EUROPEAN SEARCH REPORT
ON EUROPEAN PATENT APPLICATION NO.**

EP 20 15 0307

This annex lists the patent family members relating to the patent documents cited in the above-mentioned European search report.
The members are as contained in the European Patent Office EDP file on
The European Patent Office is in no way liable for these particulars which are merely given for the purpose of information.

14-07-2020

| Patent document cited in search report | Publication date | Patent family member(s) | Publication date |
|---|---|---|---|
| WO 2018220600 A1 | 06-12-2018 | US 2020161000 A1<br>WO 2018220600 A1 | 21-05-2020<br>06-12-2018 |

EPO FORM P0459

For more details about this annex : see Official Journal of the European Patent Office, No. 12/82

13